# EUROPEAN PATENT SPECIFICATION

(11) **EP 1 804 871 B1**
(45) Date of publication and mention of the grant of the patent: **20.06.2012**
(21) Application number: 05794138.7
(22) Date of filing: 17.10.2005
(51) Int. Cl.: A61M 15/00

(54) **An improved spacer**
Verbessertes Abstandsglied
Dispositif pour aerosols amélioré

(30) Priority: 15.10.2004 IN MU11132004
(43) Date of publication of application: 11.07.2007
(73) Proprietor: Cipla Ltd., Mumbai 400 008 (IN)
(72) Inventor: LULLA, Amar, 131 Maker Towers "L", Mumbai 400 005, Maharashtra (IN); RAO, Xerxes, Flat No. 802, Moraba Mansion, Mumbai 400 052, Maharashtra (IN)
(74) Representative: Turner, Craig Robert
(86) International application number: PCT/GB2005/003984
(87) International publication number: WO 2006/040585

(56) References cited:
- EP-A- 0 938 908
- EP-A2- 0 347 779
- WO-A-00/33902
- WO-A2-2008/039925
- GB-A- 2 230 456
- US-A- 4 449 525
- US-A- 4 592 348
- US-A- 5 042 467
- US-A1- 2002 005 195
- US-A1- 2004 123 974
- ANONYMOUS: "Butterfly valve" INTERNET ARTICLE, [Online] XP002341637 Retrieved from the Internet: URL:HTTP://www.yourdictionnary.com/ahd/b/b 058220.html> [retrieved on 2005-08-22]

## Description

The present invention relates to a spacer device for the oral administration of a volatile medium containing a medicament.

Spacer devices are attachments to the mouthpiece of an inhaler, particularly for pressurized meter dose inhalers. Various spacer devices are known in the art from a tube spacer with a volume of < 50ml to holding chambers with a volume of up to 750 ml. Generally, spacers are known to reduce coordination difficulties and reduce oropharyngeal deposition thereby considerably increasing the drug delivery in the lungs.

Indian Patent No. 190657 discloses a spacer device for administering orally a volatile liquid composition by inhalation having two conical members made of anti-static material and assembled at their divergent ends. Convergent end of the cone is adapted to receive within it a pumping device of a container filled within the medicinal composition and the convergent end of the other cone adapted to be inserted into the mouth of a patient. The inner surface of the one of said cones is provided with stepped rings and the outer surface of the other cone provided with stepped rings corresponding to the stepped rings in the inner surface. The cones are provided with locking means, such as notch and the projection. Reference can also be made to WO 00/33902.

However, in the spacer device as described in the Indian Patent No. 190657 during accidental exhalation the dose in the spacer chamber gets diluted with the moist breath thereby reducing the efficiency of the drug delivery.

EP 938 908 discloses a cloud chamber (or spacer) having a one-way inspiratory air and one-way expiratory air valving system, which allows repeated breaths to be taken without removal of the device from the mouth of the user. The one-way inspiratory air valve is formed from an elliptically-shaped valve body comprising an elastomeric material having an "X"-shaped cut in the centre portion of the material. During inspiration, the "X"-shaped cut opens, allowing air to be drawn through. US 5,042,467 discloses a spacer having a similar type of valve. However, this document is primarily concerned with providing a medication inhaler incorporating a sonic signalling device comprising an integrally molded plastic body and vibratory reed. A musical tone alerts the user if he is inhaling too rapidly.

An inhalation chamber for use with a metered-dose inhaler is also disclosed in GB 2230456. A face mask adapted to communicate with the nose and/or mouth of an infant or young child communicates with the chamber outlet via a first inhalation valve, and communicates with atmosphere via a second valve permitting exhalation there through. The inhalation valve comprises a disc which is biased into a closed position in which it bears against an annular seat by means of a spring. The spring is trapped between the disk and a pair of cross-wires.

US2002/005195 discloses a type of umbrella valve.

US4592348 discloses a valve comprising a single flap.

We have now appreciated that the spacers described in the prior art and those commercially available to date, whilst of some merit, are not entirely satisfactory. In particular, spacers comprising inhalation valves of the elastomeric slit membrane type have the disadvantage that the valve may not open sufficiently, particularly if the spacer is being used by an infant or young child. In such a valve there is variable opening of the valve depending on the inspiratory airflow.

We have now appreciated the need for an improved spacer and have devised one which substantially overcomes the problems associated with the known devices.

According to the present invention, there is provided a A spacer device (100) suitable for use with a metered dose inhaler for the oral administration of a volatile medium containing a medicament, which device comprises a chamber (110) having an inlet (102) to admit a measured dose of medicament and an outlet (104) to be received in the mouth, wherein the outlet of the spacer comprises a valve (106), characterised in that valve (106) comprises a flap arrangement consisting of two flaps (108) wherein each flap is mounted so as to pivot freely around its axis in response to inhalation of the patient

The valve acts as a barrier to exhalation.

The butterfly valve comprises two flaps pivotally mounted on a valve seat. The valve comprises two flaps, more preferably two substantially semi circular flaps. In one preferred embodiment, two flaps pivot about an axis which passes through, or close to, the centre of the flow path. Suitably, the axis is substantially perpendicular to the flow path. Preferably, each flap comprises projections, suitably two projections, which enable it to be mounted on the valve seat. The flap may, for example, rotate about an axis between two projections of the flap (as will be further appreciated from the accompanying drawings). Preferably the projections of the flaps locate in corresponding recesses in the valve seat and the flaps rotate about the projections. Preferably the projections provide the only connection or mounting point between the flaps and the remainder of the valve. Preferably, each projection and its corresponding recess is substantially cylindrical. The valve is configured so as to permit air flow in one direction only - that is, out of the chamber. Preferably, the overall shape of the valve is circular.

The spacer device of the present invention is of a simple and efficient design, and this facilitates manufacture. The use of a butterfly valve allows operation of the spacer even at very low flow rates. Efficient operation is also achieved because the valve opens completely, even at low flow rates, rather than partially. We have also found that, in the context of spacers, butterfly valves do not show increased wear and tear over other types of valves, and also have the advantage of being easier to clean in situ, particularly when used with a spacer of our preferred design. The butterfly valve preferably emits an audible sound upon operation (usually upon closure) and this provides a useful indication to the user. The audible feature can be an integral part of the functioning of the valve - that is, no separate sonic signalling device is required - so this provides a further advantage.

The valve can be made of any suitable material, for example any rigid plastic or an antistatic non-metallic material, such as an antistatic plastic material, may be used. We prefer to use an acetyl copolymer (for example Delrin). Polyamide may also be used.

It is preferred to use a butterfly valve which operates at a very low expiratory flow rate. The valve preferably operates at an expiratory air flow of less than 25 ml per minute, for example from 15 ml to 25 ml per minute, or less. This enables closure of the valve even upon slight expiration by the user, Preferably, the valve also operates at a very low inspiratory flow rate. The valve preferably operates at an inspiratory air flow rate of less than 25ml per minute, for example from 15ml to 25ml per minute, or less. Complete valve opening thus occurs even upon slight inspiration by the user.

The butterfly valve is positioned in close proximity to the outlet. Whilst the spacer device may be of various shapes and constructions, it is particularly preferred to use a spacer comprising two frustoconical members members assembled together coaxially at their divergent ends, the inlet and outlet being respectively at opposed convergent ends.

Further details of such a spacer can be obtained by reference to our publication WO 00/33902. Thus, when using such a spacer, the butterfly valve is preferably positioned at, or close to, the convergent end of the frustoconical member forming the outlet.

Preferably, the butterfly valve emits an audible sound upon closure.

The outlet of the spacer preferably comprises means for guarding the valve so as to prevent damage to the valve. Suitably, the guarding means may comprise a grid spanning the outlet although various designs are possible.

The chamber of the spacer is preferably made of an antistatic non-metallic material, for example an antistatic plastic material, for example polyamide. The valve may also be made of the same material.

For a spacer comprising two frustoconical members as described above, preferably the divergent end of one member is received in the divergent end of the other member so as to provide a substantially air-tight seal. Preferably, the divergent ends have complementary stepped surfaces to provide a close air-tight fit. It is also preferred to provide locking means to lock the two members together in assembled condition.

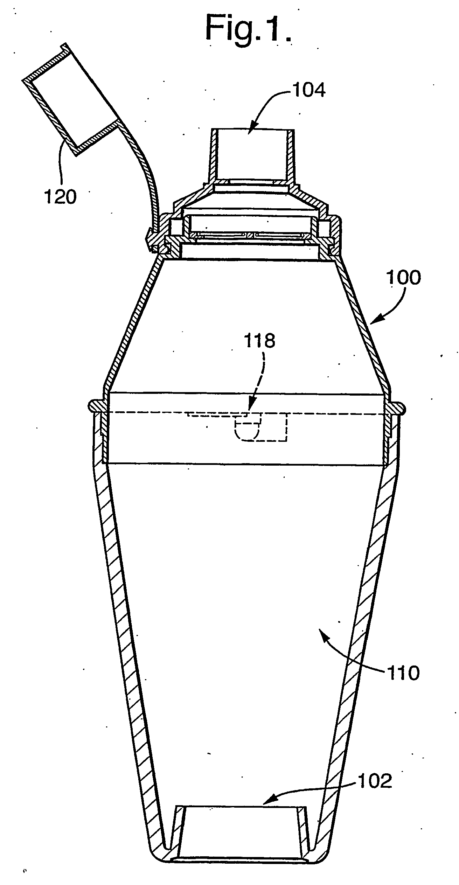

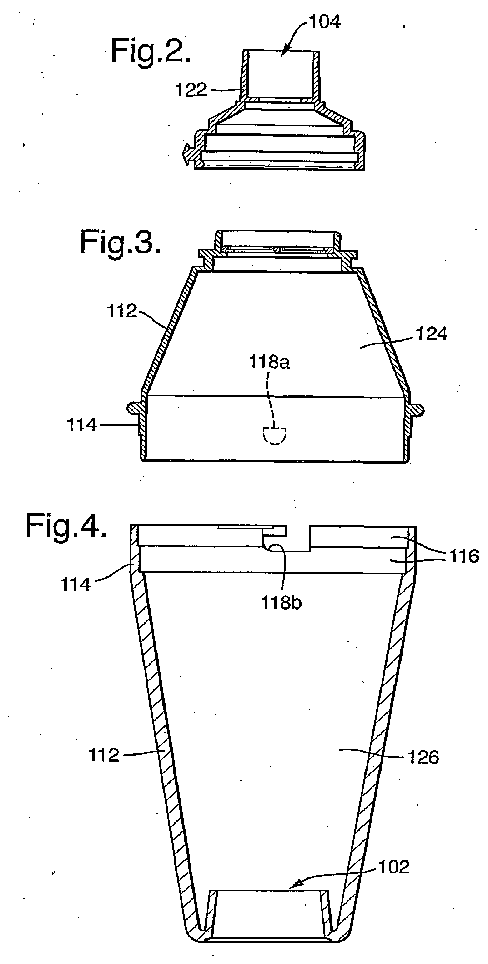

Preferably, the inlet of the spacer projects inside the chamber. For example, one embodiment of this is illustrated in Figures 1 and 4, which show inlet 102.

The spacer preferably also comprises one or more airvents. These are preferably located at the convergent end of the chamber member bearing the outlet, and allow for exit of any exhaled air to the atmosphere.

The spacer device preferably comprises an outlet having a mouthpiece. The mouthpiece preferably further comprises a cap, which may be attached or removable , as illustrated, for example, in Figure 1. The cap preferably also comprises a connecting portion which attaches to a surface of the spacer, such that even when removed from the outlet the cap remains attached to the body of the spacer device.

The invention also provides the combination of an inhaler for dispensing a measured dose of medicament in a volatile medium and a spacer device according to the present invention.

The use of a spacer device according to the present invention for the inhalation of a medicament in a volatile medium is also disclosed.

To illustrate the invention, a preferred embodiment thereof will now be described with reference to the accompanying drawings in which:
Figure 1 shows a general perspective view of a spacer device according to present invention.
Figure 2 shows a sectional view of the mouthpiece assembly of the spacer of Figure 1.
Figure 3 shows a sectional view of the top of the spacer of Figure 1.
Figure 4 shows a sectional view of the bottom of the spacer of Figure 1.
Figure 5 shows a top view of the valve.
Figure 6a shows a front view of a preferred butterfly valve; while Figure 6b shows a front view of one or the semicircular flaps used in the butterfly valve of Figure 6a.
Figure 7 shows a general perspective view of a spacer device according to the present invention.
Figure 8 shows an exploded general perspective view of the spacer device of Figure 7.
Figure 9 shows a cross-sectional view of the spacer device of Figure 8.

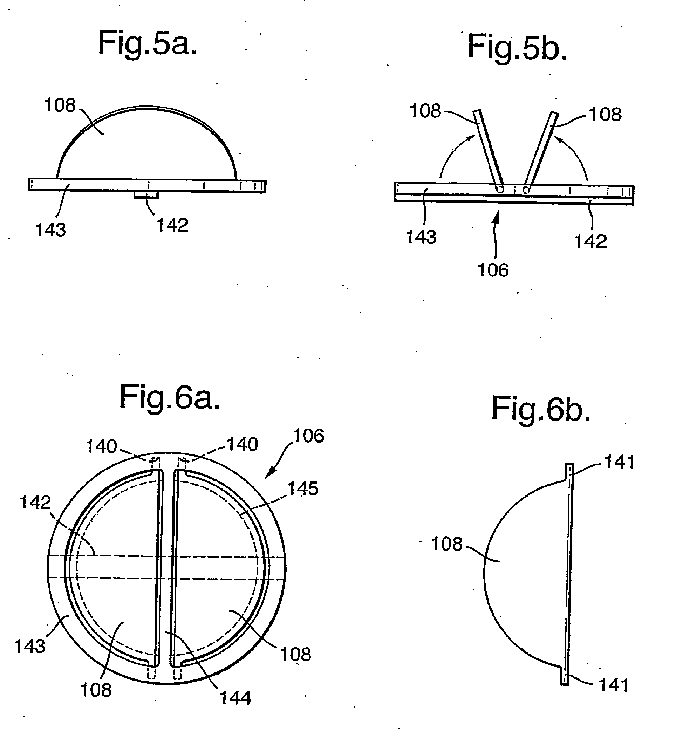

The butterfly valve (106) functions in such a manner that when the medicament is inhaled by the user, the flaps of the butterfly valve open as shown in Figure 5 and allow the medicament to pass thorough the outlet into the patient's mouth. When there is exhalation by the user into the spacer device, even at substantially low expiration airflow, the flaps (108) of the butterfly valve (106) close the outlet and act as a barrier to exhalation. This prevents the chamber-containing medicament from being diluted with the moist air. While closing the outlet, the valve creates a 'tap' sound that provides an audio feedback to the patient. The butterfly valve functions at a very low expiratory air flow of 15 to 25 ml per minute.

The chamber (110) of the spacer device (100) has two conical members named spacer top (124) and spacer bottom (126) each having a convergent (112) and a divergent end (114); said members are assembled at divergent ends (114). The divergent ends (114) of the said conical members are provided with stepped rings (116) that enables the assembly of the said conical chambers and provides an airtight joint. The inlet (102) for receiving the medicament from the inhaler or the like and outlet (104) to deliver the medicament are placed on the opposite convergent ends (112) of said conical members so as to provide substantially good drug delivery.

A locking means (118) is provided on the said conical members for locking the assembled conical members. According to the present invention, a small projection (118a) is provided on one of the conical chambers and the other conical chamber is provided with a slit (118b) that fits the projection thereby providing a lock.

Further, the spacer device is provided with a cap (120) that covers the outlet of the spacer device. The cap is preferably attached to the spacer device.

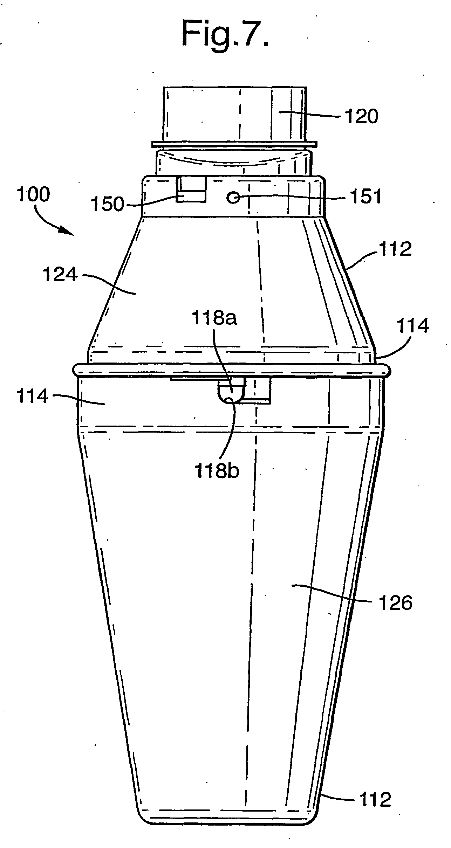

According to an embodiment of the present invention, the outlet is provided with a mouthpiece (122). The mouthpiece is covered by the cap 120 as shown in Figures 1 and 7.

The mouthpiece assembly shown generally in Figure 2 comprises the butterfly valve (106) together with associated guarding means (not shown in Figure 2).

The spacer device of the invention can be made in accordance with known techniques, as will be clear to those skilled in the art.

In use, inlet 102 is connected to an aerosol medicament reservoir, for example, and a dose of inhalant medicament is pumped into chamber 110. The patient then places outlet 104 in the mouth and inhales steadily to draw the medicament into the lungs.

Figures 6a and 6b show a preferred embodiment of the valve (106). Valve (106), comprising a valve seat (143) with central spindle (144), supports two semi circular flaps (108) having projections (141) as shown in Figure 6b. The projections (141) locate within recesses (140) in seat (143) such as to allow rotational movement of the flaps (108) (which may be better appreciated from Figure 5). Preferably, recesses (140) and corresponding projections (141) are substantially cylindrical. Each flap (108) pivots very freely on the projections (141), thus enabling operation of the valve at low flow rates. When in the closed position, the flaps (108) seal against circumferential annulus (145) which may be integrally formed with the seat (143). Cross-member (142) may, for example, optionally be provided to give structural support.

Figure 7 additionally shows airvent (150) and notch (151) which may serve as a point of attachment of the cap (120) to the device (as shown in Figure 1).

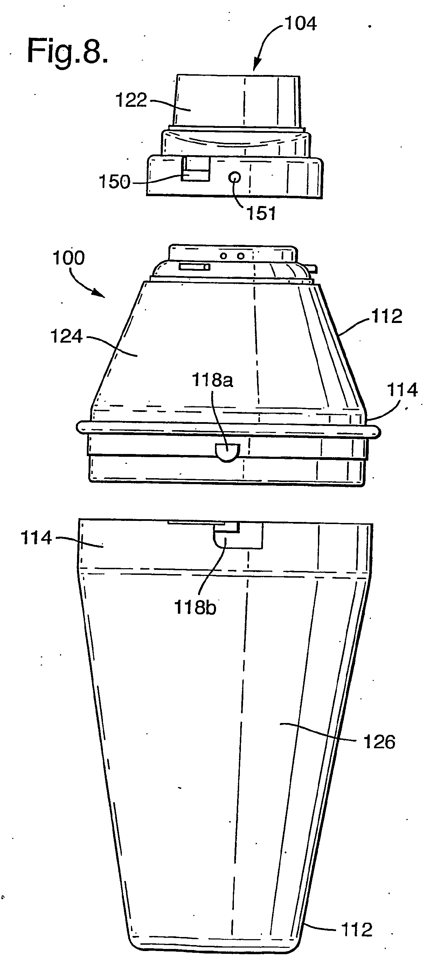

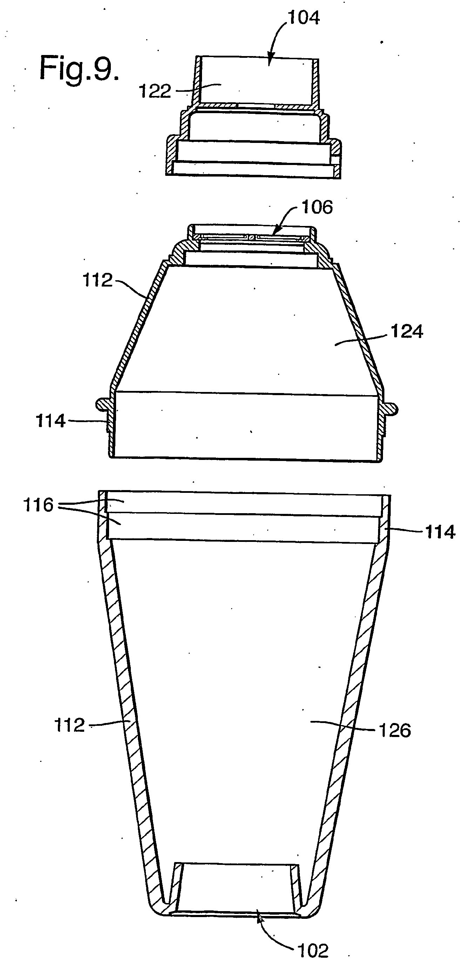

Figures 8 and 9 provide further illustration of the various features described above.

The invention is further illustrated by the following Example.

### Example 1

### FINE PARTICLE DOSE (FPD) BY CASCADE IMPACTOR USING DIFFERENT SPACERS

### (Using Flixotide evohaler-250mcg/spray.)

| | Spacer with valve According to the present invention | Aerochamber Plus spacer (Trudell Medical International) | Volumatic spacer (Glaxo) |
|---|---|---|---|
| FPD (mcg) | 112.28 | 55.18 | 12.59 |

T1/2 LIFE OF DOSE IN SPACER OF PRESENT INVENTION AND SPACER SIMILAR TO THAT OF US 5,042,467

### (Using Salbutamol inhaler)

| | Spacer with valve according to the present invention | Spacer similar to US 5,042,467 |
|---|---|---|
| FPD (mcg) after 2 sec lag time | 65.01 | 10.5 |
| FPD (mcg) after 45 sec lag time | 40.25 | 2.7 |

Lag time is the time between actuation of the dose into the spacer and dosing into the apparatus for testing (Anderson Cascade Impactor)

T1/2 of the dose in the spacer is the quantitative measure of the time up to which 50% of the respirable dose is available for inhalation. It is the time taken for reduction of the FPD value to 50% of the original. The results show the FPD value is maintained at a much higher level for longer with a spacer device according to the invention.

## Claims

1. A spacer device (100) suitable for use with a metered dose inhaler for the oral administration of a volatile medium containing a medicament, which device comprises a chamber (110) having an inlet (102) to admit a measured dose of medicament and an outlet (104) to be received in the mouth, wherein the outlet of the spacer comprises a valve (106), **characterised in that** valve (106) comprises a flap arrangement consisting of two flaps (108) wherein each flap is mounted so as to pivot freely around its axis in response to inhalation of the patient.

2. A device according to claim 1 wherein the valve operates at a low expiratory air flow of 15 to 25 ml per minute or less.

3. A device according to claim 1, or 2wherein the valve operates at a low inspiratory air flow of 15 to 25ml per minute or less,

4. A device according to claim 1, 2 or 3 wherein the valve emits an audible sound upon closure.

5. A device according to any one of claims 1 to 4 wherein the outlet comprises means for guarding the valve.

6. A device according to claim 5 wherein the means for guarding the valve comprises a grid spanning the outlet.

7. A device according to any preceding claim wherein the chamber is made of an antistatic non-metallic material.

8. A device according to any preceding claim wherein the chamber is made of an antistatic plastic material.

9. A device according to claim 8 wherein the chamber is made of polyamide.

10. A device according to any preceding claim wherein the valve is made of any rigid plastic or antistatic non-metallic material.

11. A device according to any preceding claim wherein the valve is made of an acetyl copolymer or polyamide

12. A device according to any preceding claim wherein the chamber comprises two frustoconical members assembled together coaxially at their divergent ends, said inlet and outlet being respectively at the opposed convergent ends.

13. A device according to claim 12 wherein the divergent end of one member is received in the divergent end of the other member to provide a substantially airtight seal.

14. A device according to claim 13 wherein the said divergent ends have complementary stepped surfaces to provide a close air-tight fit.

15. A device according to claim 12,13 or 14 wherein locking means are provided to lock the two members together in assembled condition.

16. A device according to any preceding claim wherein the inlet projects inside the chamber.

17. A device according to any preceding claim wherein the outlet comprises a mouthpiece.

18. A device according to claim 17 wherein the mouthpiece further comprises a cap.

19. A device according to claim 18 wherein the cap is attached to the device or is a removable cap.

20. A combination of an inhaler for dispensing a measured dose of a medicament in a volatile medium and a spacer device according to any one of claims 1 to 18.

## Patentansprüche

1. Zwischenstück-Vorrichtung (100), geeignet zur Verwendung mit einem Dosierinhalator für die orale Verabreichung eines flüchtigen Mediums, das ein Medikament enthält, die Vorrichtung umfassend eine Kammer (110) mit einem Einlass (102) zum Einlassen einer abgemessenen Dosis eines Medikaments und einen Auslass (104) zum Empfangen im Mund, wobei der Auslass des Zwischenstücks ein Ventil (106) umfasst, **dadurch gekennzeichnet, dass** das Ventil (106) eine aus zwei Klappen (108) bestehende Klappenanordnung umfasst, wobei jede Klappe montiert ist, um als Reaktion auf Inhalation des Patienten frei um ihre Achse zu schwenken.

2. Vorrichtung nach Anspruch 1, wobei das Ventil bei einem niedrigen Ausatmungs-Luftstrom von 15 bis 25 ml pro Minute oder weniger operiert.

3. Vorrichtung nach Anspruch 1 oder 2, wobei das Ventil bei einem niedrigen Einatmungs-Luftstrom von 15 bis 25 ml pro Minute oder weniger operiert.

4. Vorrichtung nach Anspruch 1, 2 oder 3, wobei das Ventil nach dem Schließen einen hörbaren Ton ausgibt.

5. Vorrichtung nach einem der Ansprüche 1 bis 4, wobei der Auslass Mittel zum Schützen des Ventils umfasst.

6. Vorrichtung nach Anspruch 5, wobei das Mittel zum Schützen des Ventils ein Gitter, das den Auslass überspannt, umfasst.

7. Vorrichtung nach einem der vorstehenden Ansprüche, wobei die Kammer aus einem antistatischen nichtmetallischen Material hergestellt ist.

8. Vorrichtung nach einem der vorstehenden Ansprüche, wobei die Kammer aus einem antistatischen Kunststoffmaterial hergestellt ist.

9. Vorrichtung nach Anspruch 8, wobei die Kammer aus Polyamid hergestellt ist.

10. Vorrichtung nach einem der vorstehenden Ansprüche, wobei das Ventil aus einem beliebigen starren Kunststoff- oder antistatischen nichtmetallischen Material hergestellt ist.

11. Vorrichtung nach einem der vorstehenden Ansprüche, wobei das Ventil aus einem Acetyl-Copolymer oder Polyamid hergestellt ist.

12. Vorrichtung nach einem der vorstehenden Ansprüche, wobei die Kammer zwei kegelstumpfförmige Glieder umfasst, die an ihren divergenten Enden koaxial zusammengebaut sind, wobei der Einlass und Auslass jeweils an den gegenüberliegenden konvergenten Enden sind.

13. Vorrichtung nach Anspruch 12, wobei das divergente Ende eines Glieds in dem divergenten Ende des anderen Glieds empfangen wird, um eine im Wesentlichen luftdichte Abdichtung bereitzustellen.

14. Vorrichtung nach Anspruch 13, wobei die divergenten Enden komplementär abgestufte Oberflächen aufweisen, um einen nahen luftdichten Sitz bereitzustellen.

15. Vorrichtung nach Anspruch 12, 13 oder 14, wobei Verriegelungsmittel bereitgestellt sind, um die zwei Glieder im zusammengebauten Zustand miteinander zu verriegeln.

16. Vorrichtung nach einem der vorstehenden Ansprüche, wobei der Einlass im Inneren der Kammer hervorragt.

17. Vorrichtung nach einem der vorstehenden Ansprüche, wobei der Auslass ein Mundstück umfasst.

18. Vorrichtung nach Anspruch 17, wobei das Mundstück weiterhin eine Kappe umfasst.

19. Vorrichtung nach Anspruch 18, wobei die Kappe an der Vorrichtung angebracht ist oder eine entfernbare Kappe ist.

20. Kombination eines Inhalators zum Abgeben einer abgemessenen Dosis eines Medikaments in einem flüchtigen Medium und einer Zwischenstück-Vorrichtung nach einem der Ansprüche 1 bis 18.

## Revendications

1. Dispositif espaceur (100) convenant à une utilisation avec un inhalateur-doseur pour l'administration par voie orale d'une substance volatile contenant un médicament, ce dispositif comprenant une chambre (110) avec un orifice d'admission (102) pour recevoir une dose mesurée de médicament et un orifice de sortie (104) destiné à être reçu dans la bouche, cas dans lequel l'orifice de sortie de l'espaceur comporte une soupape (106), **caractérisé en ce que** la soupape (106) comporte un agencement à volet consistant en deux volets (108), chaque volet étant monté de sorte à pivoter librement autour de son axe en réaction à une inhalation par le patient.

2. Dispositif selon la revendication 1, la soupape fonctionnant à un faible débit d'expiration de l'air de 15 à 25 ml/minute ou moins.

3. Dispositif selon la revendication 1 ou 2, la soupape fonctionnant à un faible débit d'inspiration de l'air de 15 à 25 ml/minute ou moins.

4. Dispositif selon la revendication 1, 2 ou 3, la soupape produisant un son audible au moment de sa fermeture.

5. Dispositif selon l'une quelconque des revendications 1 à 4, l'orifice de sortie comportant des moyens pour protéger la soupape.

6. Dispositif selon la revendication 5, les moyens pour protéger la soupape comportant une grille laquelle recouvre l'orifice de sortie.

7. Dispositif selon l'une quelconque des revendications précédentes, la chambre étant réalisée en une matière non métallique antistatique.

8. Dispositif selon l'une quelconque des revendications précédentes, la chambre étant réalisée en une matière plastique antistatique.

9. Dispositif selon la revendication 8, la chambre étant réalisée en polyamide.

10. Dispositif selon l'une quelconque des revendications précédentes, la soupape étant réalisée en n'importe quelle matière plastique rigide ou non métallique antistatique.

11. Dispositif selon l'une quelconque des revendications précédentes, la soupape étant réalisée en acétyl copolymère ou en polyamide.

12. Dispositif selon l'une quelconque des revendications précédentes, la chambre comportant deux éléments frusto-coniques assemblés coaxialement l'un à l'autre au niveau de leurs extrémités divergentes, lesdits orifice d'admission et orifice de sortie se situant respectivement au niveau des extrémités convergentes opposées.

13. Dispositif selon la revendication 12, l'extrémité divergente de l'un des éléments étant reçue dans l'extrémité divergente de l'autre élément afin de procurer un joint sensiblement étanche à l'air.

14. Dispositif selon la revendication 13, lesdites extrémités divergentes possédant des surfaces complémentaires en gradins afin de procurer un ajustage serré étanche à l'air.

15. Dispositif selon la revendication 12, 13 ou 14, des moyens de verrouillage étant prévus pour verrouiller les deux éléments l'un à l'autre dans un état assemblé.

16. Dispositif selon l'une quelconque des revendications précédentes, l'orifice d'admission faisant saillie à l'intérieur de la chambre.

17. Dispositif selon l'une quelconque des revendications précédentes, l'orifice de sortie comportant un embout buccal.

18. Dispositif selon la revendication 17, l'embout buccal comprenant en outre un capuchon.

19. Dispositif selon la revendication 18, le capuchon étant attaché au dispositif ou étant un capuchon amovible.

20. Combinaison composée d'un inhalateur pour distribuer une dose mesurée d'un médicament présent dans une substance volatile, et d'un dispositif espaceur selon l'une quelconque des revendications 1 à 18.
